# EUROPÄISCHE PATENTSCHRIFT

(11) **EP 2 806 902 B1**
(45) Veröffentlichungstag und Bekanntmachung des Hinweises auf die Patenterteilung: **19.10.2022**
(21) Anmeldenummer: 13705386.4
(22) Anmeldetag: 15.01.2013
(51) Int. Cl.: A61L 2/20, A61B 1/12, A61L 2/24

(54) **Verfahren zur Sterilisation eines medizinischen Schlauchsets, System bestehend aus Sterilisationsvorrichtung und medizinischem Schlauchset, sowie Verwendung des Systems**
Process for sterilization of a medical hose set, system comprising as sterilisation device and a medical hose set, and use of the system
Procédure de stérilisation d'un set de tubes médicaux, système composé d'un dispositif de stérilisation et d'un set de tubes médicaux, et utilisation du système

(30) Priorität: 27.01.2012 DE 102012001566; 27.01.2012 US 201261591414 P
(43) Veröffentlichungstag der Anmeldung: 03.12.2014
(73) Patentinhaber: Fresenius Medical Care Deutschland GmbH, 61352 Bad Homburg (DE)
(72) Erfinder: FEHR, Thorsten, 66679 Losheim am See (DE); KUGELMANN, Franz, 66606 St. Wendel (DE); VEIT, Tobias, 66649 Oberthal (DE)
(74) Vertreter: Herrmann, Uwe
(86) Internationale Anmeldenummer: PCT/EP2013/000105
(87) Internationale Veröffentlichungsnummer: WO 2013/110438

(56) Entgegenhaltungen:
- DE-A1- 3 825 573
- GB-A- 2 105 591
- KR-B1- 101 103 758
- US-A- 4 552 721
- US-A- 5 556 607
- US-A- 5 869 000
- US-A- 5 871 692
- US-A1- 2005 260 097

## Beschreibung

Die vorliegende Erfindung betrifft ein Verfahren zur Sterilisation wenigstens eines Gegenstandes, ein System umfassend wenigstens eine Sterilisationsvorrichtung und einen zu sterilisierenden Gegenstand sowie die Verwendung von diesem System.

Aus dem Stand der Technik sind bereits wie beispielsweise aus der EP 1 175 230 B1 Sterilisationsverfahren unter Einsatz von Ozon bekannt, bei denen Blutschlauchsysteme in Umverpackungen in einer Sterilisationskammer mit Prozessschritten von Evakuieren und Eingasen von Ozon als Sterilisationsmittel sterilisiert werden.

Als nachteilig hierbei erweisen sich die langen Sterilisationszeiten, die teuren Umverpackungen und die Probleme, die mit einer kontrollierten Dampfphase in der Sterilisationskammer einhergehen. Hierbei kommt es nämlich oft durch die Druckschwankungen in der Sterilisationskammer zu einer Kondensation und Austrocknung, was eine definierte Sterilisationsatmosphäre in der Sterilisationskammer behindert.

Darüber sind aus dem Stand der Technik Verfahren bekannt, bei denen das Sterilisationsmittel durch Vorrichtungen mit schwer zugänglichen Sterilisationszonen hindurch geleitet wird. So ist beispielsweise aus der US 4,943,414 ein Gefäß mit einem verdampfbaren Sterilisationsmittel an das Lumen einer derartigen Vorrichtung angeschlossen, wobei anschließend das Sterilisationsmittel durch Anlegen eines Unterdruckes durch das Lumen geleitet wird.

Derartige Verfahren werden auch häufig zur Sterilisation von beispielsweise Endoskopen und ähnlichen Vorrichtungen verwendet. Da derartige Vorrichtungen sowohl innen als auch außen steril sein müssen, wird hier nur ein Ende des Hohlraums bzw. Lumens an eine Strömungsmittelleitung des Sterilisationsmittel angeschlossen, wie dies beispielsweise aus der DE 198 27 442 A1 hervorgeht, so dass das Sterilisationsmittel nach Durchtritt durch das Lumen auch das äußere der Vorrichtung sterilisieren kann.

Darüber hinaus ist es beispielsweise aus dem Stand der Technik bekannt, Umverpackungen für zu sterilisierende Vorrichtungen dadurch einzusparen, indem derartige Vorrichtungen wie Schlauch- bzw. medizinische Kassettensysteme unter sterilen Bedingungen in der Sterilisationskammer mittels Verschlusskappen verschlossen werden. Beispiele für derartige Verschlusskappen sind beispielsweise aus der DE 28 18 146 A1, der DE 38 25 573 A1 und der DE 35 15 665 C1 bekannt.

US 5 556 607 A betrifft ein Verfahren zur Sterilisation von Gegenständen unter Verwendung eines Mehrkomponenten-Dampfphasensterilisators, bei dem eine Komponente Wasser ist.

US 5 871 692 A offenbart ein Verfahren zum Reinigen, Dekontaminieren und Sterilisieren von Kathetern unter Verwendung einer Kombination von Flüssigkeits- und Gas/Plasma-Sterilisationstechniken, um die vollständige und effiziente Sterilisation eines Katheters sicherzustellen.

US 5 869 000 A offenbart eine Vorrichtung und ein Verfahren zur Zuführung eines hohen Dampfsterilisationsmittelstroms in und durch eine Lumenvorrichtung mit mindestens zwei Öffnungen und einem Strömungsweg dazwischen während des Sterilisationsprozesses.

US 4 552 721 A betrifft ein Verfahren und eine Vorrichtung zum Desinfizieren von Bluttrennvorrichtungen zur Wiederverwendung.

US 2005/260097 A1 offenbart eine Sterilisationsvorrichtung zur Sterilisation von Lumengeräten mit einer gasförmigen Chemikalie.

GB 2 105 591 A betrifft ein Verfahren und eine Vorrichtung zum Sterilisieren und Lagern von Gütern, z.B. medizinischen Instrumenten, im selben Behälter.

DE 38 25 573 A1 betrifft ein Verfahren zur Erlangung einer organischen, wasserunlöslichen Stickstoffbase, ausgehend von einer wässrigen Alkalimetallbicarbonat-Lösung und dem Hydrochlorid der Base, und ein Verfahren zur Herstellung von Natriumbicarbonat.

KR 101 103 758 B1 betrifft eine Vorrichtung und ein Verfahren zur Sterilisation von Endoskopen die in Behältern angeordnet sind.

Es ist daher die Aufgabe der vorliegenden Erfindung, ein Verfahren zur Sterilisation wenigstens eines Gegenstandes, ein System umfassend wenigstens eine Sterilisationsvorrichtung und einen zu sterilisierenden Gegenstand sowie die Verwendung von diesem System in vorteilhafter Weise weiterzubilden, insbesondere dahingehend, dass Gegenstände mit schwer zugänglichen Sterilisationszonen einfach und sicher sterilisiert werden können.

Diese Aufgabe wird erfindungsgemäß gelöst durch ein Verfahren zur Sterilisation mit den Merkmalen des Anspruchs 1. Danach ist vorgesehen, dass bei einem Verfahren zur Sterilisation wenigstens eines Gegenstandes, der Gegenstand eine erste Öffnung und eine zweite Öffnung zu einem zu sterilisierenden Innenraum des Gegenstandes aufweist. Die erste Öffnung wird an eine Zuleitung für Sterilisationsmittel angeschlossen. Die zweite Öffnung wird an einer Ableitung für Sterilisationsmittel angeschlossen. Das Sterilisationsmittel, das Wasserstoffperoxid enthält oder aus Wasserstoffperxid besteht, wird durch die Zuleitung in den Innenraum und durch den Innenraum hindurch geleitet und verlässt den Innenraum durch die Ableitung. Das Wasserstoffperoxid wird in Dampfform in den Gegenstand eingeleitet. Die erste Öffnung und die zweite Öffnung werden nach Abschluss des Sterilisationsverfahrens keimdicht versiegelt, wobei die keimdichte Versiegelung durch ein Verschließen von an dem Gegenstand angebrachten Verschlussmitteln unter sterilen Bedingungen erfolgt, die während des Sterilisationsverfahrens einen Teil der ersten Öffnung und der zweiten Öffnung ausbilden, wobei der zu sterilisierende Gegenstand ein medizinisches Schlauchset, insbesondere ein Blutschlauchsystem, ist, wobei der Gegenstand für den Sterilsationsvorgang in eine vakuumfeste Aufnahmekammer eingebracht wird und wobei das Verfahren zumindest teilweise unter Vakuum durchgeführt wird.

Bei einem derartigen Gegenstand, der zu sterilisieren ist, kann es sich insbesondere um Vorrichtungen mit schwer zugänglichen Sterilisationszonen, wie beispielsweise Blutschlauchsysteme mit längeren Lumen (diese Ausgestaltung fällt unter den Schutzumfang der Ansprüche) oder Kombinationen von Blutschlauchsystemen mit Kassetten (diese Ausgestaltung fällt nicht unter den Schutzumfang der Ansprüche) oder Filtern (diese Ausgestaltung fällt nicht unter den Schutzumfang der Ansprüche) handeln.

Durch das erfindungsgemäße Verfahren ergibt sich der Vorteil, dass eine einfache und sichere Sterilisierungsmöglichkeit geschaffen wird, wobei zugleich sehr kurze Sterilisationszeiten möglich sind. Insbesondere ist es möglich, ein direktes Trocknen und Ausblasen der Restgase nach der Sterilisation zu ermöglichen. Da der Innenraum, der zu sterilisieren ist, durch den Anschluss an die Zuleitung und die Ableitung für das Sterilisationsmittel es ermöglicht, dass der gesamte Innenraum des Gegenstandes vom Sterilisationsmittel durchströmt werden kann, ist es auch möglich, unabhängig Feuchtigkeit, Temperatur und Sterilisationsagens und damit eine Einstellung der optimalen Sterilisationsbedingungen vorzunehmen. Auch ist es nicht mehr erforderlich, eine aufwändige Apparatur, wie beispielsweise eine großvolumige Vakuumkammer für das Sterilisationsverfahren einzusetzen. Eine sehr kurze Exposition des Sterilisationsmediums auf den zu sterilisierenden Gegenstand ist möglich.

Auf eine Verwendung von Ethylenoxid (ETO) kann verzichtet werden und es ist auch nicht notwendig, Gammastrahlen zur Sterilisation einzusetzen, so dass auch keine Materialschädigung in Folge der Einwirkung von Strahlen erfolgt. Darüber hinaus ermöglicht das erfindungsgemäße Verfahren zur Sterilisation, auf eine spezielle Vor- bzw. Nachbehandlung des Gegenstandes, der zu sterilisieren ist, zu verzichten.

Der Einsatz von Wasserstoffperoxid erlaubt es vorteilhafterweise, eine besonders schonende aber zugleich wirksame Sterilisation durchführen zu können. Darüber hinaus ermöglicht der Einsatz von Wasserstoffperoxid zugleich eine zügige Sterilisation des zu sterilisierenden Gegenstandes.

Es ist möglich, dass das Wasserstoffperoxid unter Umgebungsdruck verdampft und temperiert mit einem Trägermedium in den Gegenstand eingeleitet wird, wobei das Trägermedium vorzugsweise Luft, insbesondere sterile Luft ist.

Darüber hinaus ist es möglich, dass die Temperatur des Sterilisationsmittels im Bereich zwischen ca. 40°C bis ca. 100°C liegt. Die Temperatur kann dabei je nach Anwendungsbereich gewählt werden und dabei beispielsweise 40°C, 50°C, 60°C, 70°C oder 80°C betragen. Grundsätzlich ist es möglich, sämtliche Werte aus dem Bereich zwischen 40°C bis 100°C zu wählen.

Außerdem kann vorgesehen sein, dass die Verfahrensdauer des Sterilisationsverfahrens zwischen ca. 3 bis ca. 20 Minuten gewählt wird. Insbesondere kann vorgesehen sein, dass die Verfahrensdauer des Sterilisationsverfahrens bei ca. 5 Minuten oder auch darüber und vorzugsweise bei unter 20 Minuten liegt.

Durch das keimdichte Versiegeln nach Abschluss des Sterilisationsverfahren wird es vorteilhaft möglich, auf eine Umverpackung verzichten zu können. Dabei kann man sich zu Nutze machen, dass nur im Innenraum zu sterilisierende Gegenstände auf der Außenseite nicht sterilisiert werden müssen und es somit ausreichend ist, die Öffnungen des Gegenstandes nach Abschluss des Sterilisationsverfahrens keimdicht zu versiegeln. Eine derartige Versiegelung erfolgt grundsätzlich unter sterilen Bedingungen.

Außerdem ist es möglich, dass das bzw. die Verschlussmittel ein oder mehrere Verschlusskappen sind oder umfassen.

Es kann vorgesehen sein, dass die Öffnungen einen Inline-Verschluss ermöglichen, der durch einen Einschlagstopfen realisiert wird. Dieser Einschlagstopfen kann geöffnet und verschlossen sein. Während des Sterilisationsverfahrens ist der Einschlagstopfen geöffnet und wird mit Abschluss des Sterilisationsverfahrens verschlossen.

Alternativ ist es denkbar, einen Inline-Verschluss mittels eines Septums zu realisieren. Hierbei wird an Stelle der Verschlusskappe mit Einschlagstopfen ein Septum verwendet, das durch eine Injektionsnadel durchstoßen wird. So kann beispielsweise durch die Nadel der Wasserstoffperoxiddampf eingeleitet werden, wobei sich nach Abschluss des Sterilisationsverfahrens das Septum sodann eigenständig verschließen kann, wenn die Nadel herausgezogen wird.

Auch ist es denkbar, eine Sterilisationskammer mit integrierter Kappenverschlussvorrichtung vorzusehen, wobei der zu sterilisierende Gegenstand, wie beispielsweise ein Schlauchsystem an die Sterilisationseinheit angekoppelt wird und nach der Sterilisation ein Verschließen, Zudrehen der Verschlusskappe innerhalb der ebenfalls sterilisierten Ankoppeleinheit, erfolgt. Eine weitere Möglichkeit des Verschließens im Zuge der keimdichten Versiegelung des Gegenstandes kann dadurch erfolgen, dass die Verschlusskappe nach Abschluss des Sterilisationsverfahrens zugeschweißt bzw. abgeschweißt und hierdurch verschlossen wird. Alternative Verschlußmöglichkeiten können auch beispielsweise durch den Einsatz von Sterilmembranen, Schrumpfschläuchen oder dergleichen realisiert werden.

Erfindungsgemäß ist vorgesehen, dass das Verfahren zumindest teilweise unter Vakuum durchgeführt wird. Durch die teilweise Durchführung des Verfahrens unter Vakuum kann vorteilhafterweise verhindert werden, dass es zu einem Kollabieren des Gegenstandes, wie beispielsweise eines medizinischen Schlauchsystems kommen kann.

Darüber hinaus betrifft die vorliegende Erfindung ein System gemäß Anspruch 6 umfassend wenigstens eine Sterilisationsvorrichtung sowie wenigstens einen durch die Sterilisationsvorrichtung zu sterilisierenden Gegenstand, wobei die Sterilisationsvorrichtung wenigstens eine Zuleitung und wenigstens eine Ableitung für Sterilisationsmittel aufweist, wobei der Gegenstand wenigstens zwei Öffnungen zu einem zu sterilisierenden Innenraum des Gegenstandes aufweist, wobei wenigstens eine erste Öffnung an eine Zuleitung für Sterilisationsmittel angeschlossen ist und wobei die wenigstens eine zweite Öffnung an eine Ableitung für Sterilisationsmittel angeschlossen ist und wobei die Sterilisationsvorrichtung Mittel aufweist, mittels derer das Sterilisationsmittel durch die Zuleitung in den Innenraum und durch den Innenraum hindurch leitbar ist und mittels derer das Sterilisationsmittel über die Ableitung aus dem Innenraum herausleitbar ist, wobei das Sterilisationsmittel Wasserstoffperoxid enthält oder aus Wasserstoffperoxid besteht, und das Wasserstoffperoxid in Dampfform in den Gegenstand einleitbar ist. Verschlussmittel zur keimdichten Versiegelung der ersten Öffnung und der zweiten Öffnung sind vorgesehen, wobei die keimdichte Versiegelung durch ein Verschließen von an dem Gegenstand angebrachten Verschlussmitteln unter sterilen Bedingungen erfolgbar ist, die einen Teil der ersten Öffnung und der zweiten Öffnung ausbilden, wobei der zu sterilisierende Gegenstand ein medizinisches Schlauchset, insbesondere ein Blutschlauchsystem, ist, und wobei die Sterilisationsvorrichtung eine Aufnahmekammer zur Aufnahme des zu sterlisierenden Gegenstands aufweist, wobei die Aufnahmekammer mit einer Vakuumpumpe zur Evakuierung der Aufnahmekammer in Verbindung steht.

Somit ist vorgesehen, dass in dem System die Sterilisationsvorrichtung zur Sterilisation wenigstens eines Gegenstandes, wobei die Sterilisationsvorrichtung wenigstens eine Zuleitung und wenigstens eine Ableitung für Sterilisationsmittel aufweist, wobei der Gegenstand wenigstens zwei Öffnungen zu einem zu sterilisierenden Innenraum des Gegenstandes aufweist, derart beschaffen ist, dass wenigstens eine erste Öffnung an eine Zuleitung für Sterilisationsmittel anschließbar und dass die wenigstens eine zweite Öffnung an eine Ableitung für Sterilisationsmittel anschließbar ist und dass das Sterilisationsmittel durch die Zuleitung in den Innenraum und durch den Innenraum hindurchleitbar ist und wobei das Sterilisationsmittel über die Ableitung aus dem Innenraum herausleitbar ist.

Hierzu weist die Sterilisationsvorrichtung des Systems Mittel auf, mittels derer das Sterilisationsmittel durch die Zuleitung in den Innenraum und durch den Innenraum hindurch leitbar ist und mittels derer das Sterilisationsmittel über die Ableitung aus dem Innenraum herausleitbar ist. Diese Mittel können beispielsweise Pumpelemente oder sonstige Förderelemente wie eine Druckzufuhr oder Zufuhr eines Stützgases oder dergleichen sein.

Außerdem kann vorgesehen sein, dass die Sterilisationsvorrichtung des Systems wenigstens eine Überdruckzufuhr, wenigstens ein Reservoir zur Aufnahme des Sterilisationsmittels, wenigstens ein Ventilelement, wenigstens eine Trägermediumzufuhr und wenigstens ein Heizelement aufweist, mittels derer das Sterilisationsmittel, insbesondere das Wasserstoffperoxid unter Umgebungsdruck verdampfbar und temperiert mit einem Trägermedium in den Gegenstand einleitbar ist, wobei das Trägermedium vorzugsweise Luft, insbesondere sterile Luft ist und wobei die Trägermediumzufuhr vorzugsweise eine sterile Luftzufuhr ist.

Die Sterilisationsvorrichtung des Systems weist ein Steuerungs- und/oder Regelungsmittel auf, mittels dessen die Sterilisationsvorrichtung derart Steuer- und/oder regelbar ist, dass einVerfahren zur Sterillisation eines Gegenstandes durchführbar ist und/oder dass insbesondere mittels des Steuerungs- und/oder Regelungsmittels Druck und/oder Temperatur und/oder Zusammensetzungen des Sterilisationsmittels Steuer- und/oder regelbar ist.

Außerdem betrifft die vorliegende Erfindung die Verwendung eines Systems nach einem der Ansprüche 6-8 zur Sterillisation wenigstens eines Gegenstandes, bestehend aus einem medizinischen Schlauchset, insbesondere einem Blutschlauchsystem.

Weitere Einzelheiten und Vorteile der Erfindung sollen nun anhand eines in der Zeichnung dargestellten Ausführungsbeispiels näher erläutert werden.

Es zeigen:
- Figur 1:: eine schematische Ansicht des erfindungsgemäßen Systems umfassend eineSterilisationsvorrichtung mit angeschlossenem, zu sterilisierenden Schlauchset und
- Figur 2:: eine weitere schematische Ansicht der Sterilisationsvorrichtung mit Aufnahmekammer zur Aufnahme des zu sterilisierenden Gegenstandes.

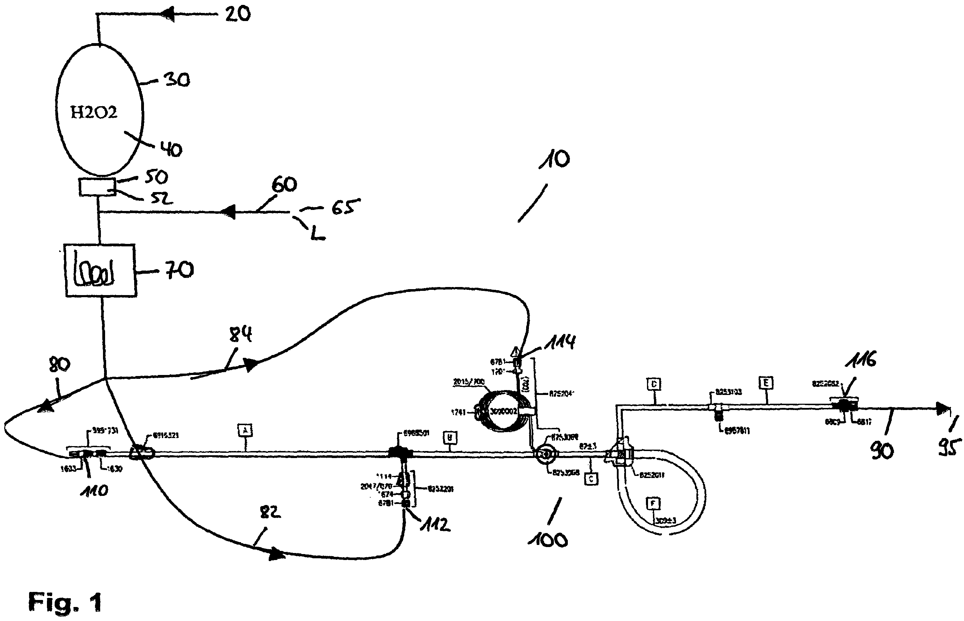

Figur 1 zeigt den schematischen Aufbau der Sterilisationsvorrichtung 10, mittels derer als zu sterilisierender Gegenstand, der eine schwer zugängliche Sterilisationszone aufweist, ein Blutschlauchsystem 100 sterilisiert wird. Die Sterilisationsvorrichtung weist dabei eine Überdruckzufuhr 20 auf, die an das Reservoir 30 zur Aufnahme des Sterilisationsmittels 40, das hier Wasserstoffperoxid ist, angeschlossen ist. Ausgangsseitig des Reservoirs 30 befindet sich als Ventilelement 50 ein Nadelventil 52, über das das Abströmen des Sterilisationsmittels geregelt werden kann.

Stromabwärts des Nadelventils 52 erfolgt der Anschluss der Trägermediumzufuhr 60, wobei als Trägermedium 65 hier sterile Luft L zugeführt wird. Stromabwärts der Trägermediumzufuhr 60 befindet sich eine Heizung 70, so dass das an dieser Stelle in Dampfform vorliegende Wasserstoffperoxid temperiert mit der sterilen Luft als Trägermedium in den Gegenstand 100 eingeleitet werden kann. Dabei liegt die Temperatur des Sterilisationsmittels im bevorzugten Bereich von ca. 40°C bis ca. 80°C, hier bei ca. 60°C. Über die Zuleitungen 80, 82, 84 wird das Sterilisationsmittel zu den Öffnungen 110, 112 und 114 des medizinischen Schlauchsets 100, das zu sterilisieren ist, zugeleitet, so dass das Sterilisationsmedium sämtliche Teile des Lumens des Schlauchsets 100 durchströmen kann. Über die Öffnung 116 verlässt das Sterilisationsmittel das Schlauchset und strömt über die Leitung 90 in den Kondensator 95 der Sterilisationsvorrichtung 10.

Das Sterilisationsverfahren läuft folglich derart ab, dass das Wasserstoffperoxid (H2O2) verdampft wird und temperiert mit dem Trägermedium, nämlich der zugeführten sterilen Luft L durch den zu sterilisierenden Gegenstand 100, hier also das Blutschlauchsystem 100 hindurch geleitet wird.

Grundsätzlich ist auch denkbar (jedoch fallen diese Ausgestaltungsformen nicht unter den Schutzumfang der Ansprüche), dass als zu sterilisierender Gegenstand jeglicher Gegenstand, der schwer zugängliche Sterilisationszonen aufweist, etwa ein Dialysefilter oder ein medizinisches Kassettensystem mit integriertem Blutschlauchsystem, mit dem Sterilisationsverfahren bzw. der Sterilisationsvorrichtung 10 sterilisiert werden kann.

Bereits nach weniger als 20 Minuten, vorzugsweise nach weniger als 5 Minuten, was jedoch von der Beschaffenheit des zu sterilisierenden Gegenstandes abhängt, kann bei einem Durchströmen des Gegenstandes 100 mit einem 35%-igen Wasserstoffperoxid-Dampf eine Sterilisation dieses Gegenstandes, z. B. eines Blutschlauchsystems 100 erreicht werden.

Die Blutschlauchsysteme 100 werden nach dem Durchströmen mit Wasserstoffperoxid-Dampf in-line verschlossen, so dass die Systeme innen hermetisch und steril verschlossen sind.

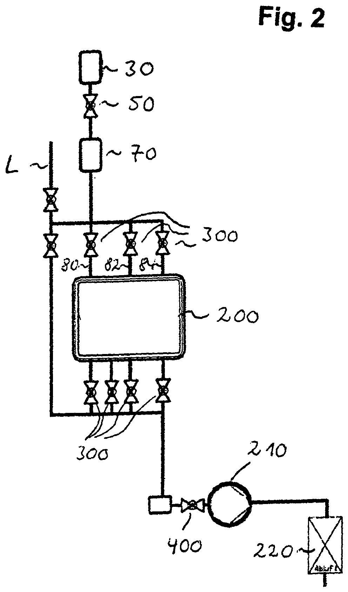

Für das beschriebene Verfahren kann die aus Figur 2 ersichtliche Anordnung Verwendung finden, in der gleiche oder funktionsgleiche Bauteile mit identischen Bezugszeichen versehen sind, wie in Figur 1.

In Figur 2 ist zusätzlich die Aufnahmekammer 200 dargestellt, die zur Aufnahme des zu sterilisierenden Gegenstandes, wie beipsielsweise eines Blutschlauchsystems 100 dient. Das Bezugszeichen 210 kennzeichnet eine Vakuumpumpe und das Bezugszeichen 220 einen Destructor für den Wasserstoffperoxid-Dampf mit Abluftanschluss.

Wie dies auf Figur 2 hervorgeht, befinden sich in Strömungsrichtung des Wasserstoffperoxid-Dampfes vor und nach der Aufnahmekammer 200 Ventile 300, die jeweils einzeln verschlossen und geöffnet werden können und durch eine nicht dargestellte Steuer- oder Regeleinheit angesteuert werden können. Des Weiteren ist ein Ventil 400 unmittelbar stromaufwärts der Vakuumpumpe 210 angeordnet.

Das Bezugszeichen L kennzeichnet die Option Warmluft. Durch das entsprechende, mittels eines oder mehrerer Ventile absperrbare Leitungssystem kann Warmluft durch das Leitungssystem stromaufwärts und stromabwärts der Aufnahmekammer 200, durch die Aufnahmekammer 200 selbst sowie auch durch das vor und nach der Vakuumpumpe 210 befindliche Leitungssystem und durch die Vakuumpumpe 210 selbst geführt werden. Die Warmluft kann als Trägermedium für Wasserstoffperoxid verwendet werden.

In einer bevorzugten Ausführungsform der Erfindung gestaltet sich der Sterilisationsvorgang wie folgt:
Das zu sterilisierende Schlauchsystem wird vorzugsweise mit allen seinen Ein- bzw. Ausgängen an die Sterilisationsvorrichtung, die beispielsweise in Figur 2 gezeigt ist, angeschlossen. Bevorzugt ist es, wenn genau oder in etwa gleich viele Schlauchenden als Eingänge und als Ausgänge für das Sterilisationsmittel benutzt werden.

Zu Beginn des Prozesses ist das Ventil 50 zwischen der Wasserstoffperoxidvorlage 30 und dem Rest des Systems geschlossen. Alle anderen Ventile sind offen. Mittels der Vakuumpumpe 210 wird das Gesamtsystem und somit auch die Aufnahmekammer 200 sowie der darin befindliche zu sterilisierende Gegenstand evakuiert. Um ein Kollabieren der flexiblen Schlauchsysteme zu verhindern, muss die Aufnahmekammer 200 ebenfalls evakuiert werden. Vorzugsweise ist die Aufnahmekammer 200 kleiner und einfacher aufgebaut als aus dem Stand der Technik bekannte Vakuumkammern.

Im nächsten Schritt wird das Ventil 50 zwischen der Wasserstoffperoxidvorlage 30 und dem Rest des Systems geöffnet. Wasserstoffperoxid wird am Verdampfer 70 verdampft und kann über die Leitungen 80, 82, 84 in das in der Aufnahmekammer 200 befindliche, in Figur 2 nicht dargestellte Schlauchsystem einströmen. Durch unterschiedliche Schaltungen der Ventile 300 an der Aufnahmekammer 200 ist es möglich, ganz gezielt spezielle Schlauchsequenzen bzw. Sequenzen des zu sterilisierenden Gegenstandes zu durchströmen. Dies ist insbesondere bei Schlauchsystemen besonders vorteilhaft, da in solchen Schlauchsystemen Schläuche mit stark unterschiedlichen Lumendurchmessern enthalten sind. Ein solches Vorgehen vergrößert sich Sicherheit des Verfahrens.

Gemäß der vorliegenden Erfindung können die für das Sterilisationsverfahren optimalen Bedingungen hinsichtlich Temperatur, Druck und Sterilisationsmedium voneinander unabhängig voneinander mittels der nicht näher dargestellten Steuerungs- und Regelungseinheit der Sterilisationsvorrichtung, die den Sterilisationsvorgang steuert und regelt, gesteuert werden.

Durch die Verwendung des Vakuums kann das Wasserstoffperoxid bei niedrigen Temperaturen verdampfen als bei Umgebungsdruck. Wird ein Trägergas verwendet, müssen höhere Temperaturen verwendet werden.

Aufgrund der sehr kurzen Zykluszeiten, von vorzugsweise bis zu 20 min. ist es möglich, das Sterilisationsverfahren als in-line Sterilisationsverfahren, also beispielsweise als Bestandteil einer Fertigungsstraße einzusetzen. So ist es denkbar, dass bei einem derartigen in-line Sterilisationsverfahren ggf. direkt in der Siegelkammer einer Umverpackungsmaschine die Blutschlauchsysteme sterilisiert und zusammen mit der Umverpackung verpackt werden.

Vorzugsweise wird bei der vorliegenden Erfindung die Umverpackung erst nach der Sterilisiation eingesetzt. Bei normalen Sterilisationsverfahren stellt die Umverpackung eine Barriere für das Sterilisationsmittel dar und erschwert und verlängert das Verfahren.

Als mögliche Ausführungsformen können lineare Systeme mit mehreren Positionen oder bevorzugt ein sogenannter "Sterilisationsrundläufer" eingesetzt werden. Bei einem derartigen Sterilisationsrundläufer können die erforderlichen Schaltzustände mit Hilfe einer Gleitringdichtung in Verbindung mit der Drehung des Rundläufers realisiert werden.

Grundsätzlich ist eine aufwändige Apparatur, die zudem eine Vakuumkammer aufweist, nicht erforderlich. Eine Vakuumkammer bzw. eine einfacher aufgebaute Aufnahmekammer kann aber vorzugsweise vorgesehen sein, um einfacher ein Kollabieren z. B. von Schlauchteile, Kammern oder Ähnlichem zu vermeiden. Im erfindungsgemäßen Verfahren ist eine solche Vakuumkammer vorgesehen.

Durch das direkte Einleiten des Wasserstoffperoxids in die Lumen der Schlauchsysteme lassen sich sehr kurze Sterilisationszeiten erreichen. Bevor die Schlauchsysteme nach der Sterilisation entnommen werden, werden diese unter sterilen Bedingungen verschlossen.

Hierzu gibt es unterschiedliche Möglichkeiten einer keimdichten Versiegelung, wie nachstehend erläutert werden soll:
Eine erste Alternative besteht darin, dass die Öffnungen einen Inline-Verschluss ermöglichen, der durch einen Einschlagstopfen realisiert wird. Dieser Einschlagstopfen kann geöffnet und verschlossen sein. Der Einschlagstopfen ist dabei beispielsweise in einer Verschlusskappe angeordnet, die auf einem Konnektor des Schlauchsystems dichtend aufgedreht bzw. aufgesetzt ist und zur Konnektierung des Konnektors des Schlauchsystems abgedreht werden kann. Während des Sterilisationsverfahrens ist der Einschlagstopfen und damit auch die Verschlusskappe geöffnet und erst mit Abschluss des Sterilisationsverfahrens wird der Einschlagstopfen in die Verschlussposition überführt.

Eine zweite Alternative ist, einen Inline-Verschluss mittels eines Septums zu realisieren. Hierbei wird an Stelle der Verschlusskappe mit Einschlagstopfen ein Septum verwendet, das durch eine Injektionsnadel durchstoßen wird. So kann beispielsweise durch die Nadel der Wasserstoffperoxiddampf eingeleitet werden, wobei sich nach Abschluss des Sterilisationsverfahrens das Septum sodann eigenständig verschließen kann, wenn die Nadel herausgezogen wird.

Eine dritte Alternative ist, eine Sterilisationskammer mit integrierter Kappenverschlussvorrichtung vorzusehen, wobei der zu sterilisierende Gegenstand, wie beispielsweise ein Schlauchsystem an die Sterilisationseinheit angekoppelt wird und nach der Sterilisation ein Verschließen, Zudrehen der Verschlusskappe innerhalb der mit sterilisierten Ankoppeleinheit, erfolgt.

Eine weitere Möglichkeit des Verschließens im Zuge der keimdichten Versiegelung des Gegenstandes kann dadurch erfolgen, dass die Verschlusskappe, z. B. ein Schlauchstück der Verschlusskappe, nach Abschluss des Sterilisationsverfahrens zugeschweißt bzw. abgeschweißt und hierdurch verschlossen wird.

Weitere alternative Verschlussmöglichkeiten können auch beispielsweise durch den Einsatz von Sterilmembranen, Schrumpfschläuchen oder dergleichen realisiert werden.

## Patentansprüche

1. Verfahren zur Sterilisation eines Gegenstandes (100), wobei der Gegenstand (100) eine erste Öffnung (110, 112, 114) und eine zweite Öffnung (116) zu einem zu sterilisierenden Innenraum aufweist, wobei in dem Verfahren:
die erste Öffnung (110, 112, 114) an eine Zuleitung (80, 82, 84) für Sterilisationsmittel (40) und die zweite Öffnung (116) an eine Ableitung (90) für Sterilisationsmittel (40) angeschlossen wird,
das Sterilisationsmittel (40), das Wasserstoffperoxid enthält oder aus Wasserstoffperoxid besteht, durch die Zuleitung (80, 82, 84) in den Innenraum und durch den Innenraum hindurch geleitet wird und den Innenraum durch die Ableitung (90) verlässt, und
das Wasserstoffperoxid in Dampfform in den Gegenstand (100) eingeleitet wird,
**dadurch gekennzeichnet, dass**
die erste Öffnung (110, 112, 114) und die zweite Öffnung (116) nach Abschluss des Sterilisationsverfahrens keimdicht versiegelt werden, wobei
die keimdichte Versiegelung durch ein Verschließen von an dem Gegenstand angebrachten Verschlussmitteln unter sterilen Bedingungen erfolgt, die während des Sterilisationsverfahrens einen Teil der ersten Öffnung (110, 112, 114) und der zweiten Öffnung (116) ausbilden,
wobei der zu sterilisierende Gegenstand (100) ein medizinisches Schlauchset, insbesondere ein Blutschlauchsystem, ist,
wobei der Gegenstand (100) für den Sterilsationsvorgang in eine vakuumfeste Aufnahmekammer (200) eingebracht wird und wobei das Verfahren zumindest teilweise unter Vakuum durchgeführt wird.

2. Verfahren nach Anspruch 1, **dadurch gekennzeichnet, dass** das Wasserstoffperoxid unter Umgebungsdruck verdampft und temperiert mit einem Trägermedium in den Gegenstand (100) eingeleitet wird, wobei das Trägermedium vorzugsweise Luft, insbesondere sterile Luft (L) ist.

3. Verfahren nach einem der vorhergehenden Ansprüche, **dadurch gekennzeichnet, dass** die Temperatur des Sterilisationsmittels (40) im Bereich zwischen 40°C bis 100°C liegt.

4. Verfahren nach einem der vorhergehenden Ansprüche, **dadurch gekennzeichnet, dass** die Verfahrensdauer zwischen 3 bis 20 Minuten, insbesondere bei 5 Minuten liegt.

5. Verfahren nach Anspruch 4, **dadurch gekennzeichnet, dass** das bzw. die Verschlussmittel ein oder mehrere Verschlusskappen sind oder umfassen.

6. System umfassend wenigstens eine Sterilisationsvorrichtung (10) sowie wenigstens einen durch die Sterilisationsvorrichtung (10) zu sterilisierenden Gegenstand (100), wobei die Sterilisationsvorrichtung (10) wenigstens eine Zuleitung (80, 82, 84) und wenigstens eine Ableitung (90) für Sterilisationsmittel (40) aufweist, wobei der Gegenstand (100) wenigstens zwei Öffnungen (110, 112, 114, 116) zu einem zu sterilisierenden Innenraum des Gegenstandes (100) aufweist, wobei wenigstens eine erste Öffnung (110, 112, 114) an eine Zuleitung (80, 82, 84) für Sterilisationsmittel (40) angeschlossen ist und wobei die wenigstens eine zweite Öffnung (116) an eine Ableitung (90) für Sterilisationsmittel (40) angeschlossen ist und wobei die Sterilisationsvorrichtung (10) Mittel aufweist, mittels derer das Sterilisationsmittel (40) durch die Zuleitung (80, 82, 84) in den Innenraum und durch den Innenraum hindurch leitbar ist und mittels derer das Sterilisationsmittel (40) über die Ableitung (90) aus dem Innenraum herausleitbar ist, wobei das Sterilisationsmittel Wasserstoffperoxid enthält oder aus Wasserstoffperoxid besteht, und das Wasserstoffperoxid in Dampfform in den Gegenstand (100) einleitbar ist,
**dadurch gekennzeichnet, dass**
Verschlussmittel zur keimdichten Versiegelung der ersten Öffnung (110, 112, 114) und der zweiten Öffnung (116) vorgesehen sind, wobei die keimdichte Versiegelung durch ein Verschließen von an dem Gegenstand angebrachten Verschlussmitteln unter sterilen Bedingungen erfolgbar ist, die einen Teil der ersten Öffnung (110, 112, 114) und der zweiten Öffnung (116) ausbilden,
dass der zu sterilisierende Gegenstand (100) ein medizinisches Schlauchset, insbesondere ein Blutschlauchsystem, ist,
und dass die Sterilisationsvorrichtung eine Aufnahmekammer (200) zur Aufnahme des zu sterlisierenden Gegenstands (100) aufweist, wobei die Aufnahmekammer (200) mit einer Vakuumpumpe (210) zur Evakuierung der Aufnahmekammer (200) in Verbindung steht.

7. System nach Anspruch 6,
**dadurch gekennzeichnet, dass** die Sterilisationsvorrichtung (10) wenigstens eine Überdruckzufuhr (20), wenigstens ein Reservoir (30) zur Aufnahme des Sterilisationsmittels (40), wenigstens ein Ventilelement (50), wenigstens eine Trägermediumzufuhr (60) und wenigstens ein Heizelement (70) aufweist, mittels derer das Sterilisationsmittel (40), insbesondere das Wasserstoffperoxid, unter Umgebungsdruck verdampfbar und temperiert mit einem Trägermedium in den Gegenstand einleitbar ist, wobei das Trägermedium vorzugsweise Luft, insbesondere sterile Luft (L) ist und wobei die Trägermediumzufuhr (60) vorzugsweise eine sterile Luftzufuhr ist.

8. System nach Anspruch 6 oder 7,
**dadurch gekennzeichnet, dass** mit der Sterilisationsvorrichtung (10) ein Verfahren nach einem der Ansprüche 1 bis 5 durchführbar ist, wobei vorzugsweise die
Sterilisationsvorrichtung (10) ein Steuerungs- und/oder Regelungsmittel aufweist, mittels dessen die Sterilisationsvorrichtung (10) derart Steuer- und/oder regelbar ist, dass das Verfahren nach einem der Ansprüche 1 bis 5 durchführbar ist und/oder
dass insbesondere mittels des Steuerungs- und/oder Regelungsmittels Druck und/oder Temperatur und/oder Zusammensetzung des Sterilisationsmittels (40) steuer- und/oder regelbar ist.

9. Verwendung eines Systems nach einem der Ansprüche 6 bis 8 zur Sterilisation wenigstens eines Gegenstandes (100), bestehend aus einem medizinischen Schlauchset, insbesondere einem Blutschlauchsystem.

## Claims

1. A method for sterilising an object (100), wherein the object (100) comprises a first opening (110, 112, 114) and a second opening (116) to an interior space to be sterilised, wherein in the method:
the first opening (110, 112, 114) is connected to a supply line (80, 82, 84) for sterilising agent (40) and the second opening (116) is connected to a discharge line (90) for sterilising agent (40),
the sterilising agent (40), which contains hydrogen peroxide or consists of hydrogen peroxide, is conveyed through the supply line (80, 82, 84) into the interior space and through the interior space and exits the interior space through the discharge line (90), and
the hydrogen peroxide is introduced into the object (100) in vapour form,
**characterised in that**
the first opening (110, 112, 114) and the second opening (116) are sealed in a germ-tight manner after completion of the sterilisation method, wherein
the germ-tight sealing is carried out by closing, under sterile conditions, closure means attached to the object, which closure means form a part of the first opening (110, 112, 114) and of the second opening (116) during the sterilisation method,
wherein the object (100) to be sterilised is a medical tubing set, more particularly a blood tubing system,
wherein for the sterilisation method, the object (100) is introduced into a vacuum-proof receiving chamber and wherein the method is carried out at least partially under vacuum.

2. A method according to claim 1, **characterised in that** the hydrogen peroxide is evaporated under ambient pressure and is introduced into the object (100) in a temperature-controlled manner with a carrier medium, wherein the carrier medium is preferably air, more particularly sterile air (L).

3. A method according to one of the preceding claims, **characterised in that** the temperature of the sterilising agent (40) is in the range between 40°C and 100°C.

4. A method according to one of the preceding claims, **characterised in that** the duration of the method is between 3 and 20 minutes, more particularly is 5 minutes.

5. A method according to claim 4, **characterised in that** the closure means is or are, or comprises or comprise, one or more closure caps.

6. A system comprising at least one sterilising device (10) and at least one object (100) to be sterilised by the sterilising device (10), wherein the sterilising device (10) comprises at least one supply line (80, 82, 84) and at least one discharge line (90) for sterilising agent, wherein the object (100) comprises at least two openings (110, 112, 114, 116) to an interior space to be sterilised of the object (100), wherein at least one first opening (110, 112, 114) is connected to a supply line (80, 82, 84) for sterilising agent (40) and wherein the at least one second opening (116) is connected to a discharge line (90) for sterilising agent (40) and wherein the sterilising device (10) comprises means by means of which the sterilising agent (40) can be conveyed through the supply line (80, 82, 84) into the interior space and through the interior space and by means of which the sterilising agent (40) can be conveyed out of the interior space through the discharge line (90), wherein the sterilising agent contains hydrogen peroxide or consists of hydrogen peroxide, and the hydrogen peroxide can be introduced into the object (100) in vapour form,
**characterised in that**
closure means are provided for the germ-tight sealing of the first opening (110, 112, 114) and of the second opening (116), wherein the germ-tight sealing can be carried out under sterile conditions by closing closure means attached to the object, which closure means form a part of the first opening (110, 112, 114) and of the second opening (116),
that the object to be sterilised (100) is a medical tubing set, more particularly a blood tubing system,
and that the sterilising device comprises a receiving chamber (200) for receiving the object (100) to be sterilised, wherein the receiving chamber (200) is in connection to a vacuum pump (210) for evacuating the receiving chamber (200).

7. A system according to claim 6, **characterised in that**
the sterilisation device (10) comprises at least one overpressure feed (20), at least one reservoir (30) for receiving the sterilising agent (40), at least one valve element (50), at least one carrier medium feed (60) and at least one heating element (70) by means of which the sterilising agent (40), more particularly the hydrogen peroxide, can be evaporated under ambient pressure and can be introduced into the object in a temperature-controlled manner with a carrier medium, wherein the carrier medium is preferably air, more particularly sterile air (L) and wherein the carrier medium feed (60) is preferably a sterile air feed.

8. A system according to claim 6 or 7, **characterised in that** with the sterilising device (10), a method according to one of claims 1 to 5 can be carried out, wherein preferably the sterilising device (10) comprises an open-loop and/or closed-loop control means by means of which the sterilising device (10) can be controlled in an open-loop and/or closed-loop manner such that the method according to one of claims 1 to 5 can be carried out and/or
that more particularly by means of the open-loop and/or closed-loop control means, pressure and/or temperature and/or composition of the sterilising agent (40) can be controlled in an open-loop and/or closed-loop manner.

9. A use of a system according to one of claims 6 to 8 for sterilising at least one object (100), consisting of a medical tubing set, more particularly a blood tubing system.

## Revendications

1. Procédé de stérilisation d'un article (100), l'article (100) présentant une première ouverture (110, 112, 114) et une deuxième ouverture (116) vers un espace intérieur à stériliser, dans lequel procédé :
la première ouverture (110, 112, 114) est raccordée à une conduite d'alimentation (80, 82, 84) pour l'agent de stérilisation (40) et la deuxième ouverture (116) est raccordée à une conduite d'évacuation (90) pour l'agent de stérilisation (40),
l'agent de stérilisation (40), qui contient du peroxyde d'hydrogène ou consiste en peroxyde d'hydrogène, est conduit dans l'espace intérieur et à travers l'espace intérieur par la conduite d'alimentation (80, 82, 84) et quitte l'espace intérieur par la conduite d'évacuation (90), et
le peroxyde d'hydrogène est introduit dans l'article (100) sous forme de vapeur,
**caractérisé en ce que**
la première ouverture (110, 112, 114) et la deuxième ouverture (116) sont scellées de manière étanche aux germes à la fin du procédé de stérilisation,
le scellement étanche aux germes étant effectué par une fermeture de moyens de fermeture disposés sur l'article dans des conditions stériles, qui, pendant le procédé de stérilisation, forment une partie de la première ouverture (110, 112, 114) et de la deuxième ouverture (116),
l'article (100) à stériliser étant un ensemble de tubes médicaux, notamment un système de tubes pour le sang,
l'article (100) étant placé dans une chambre de réception (200) résistante au vide pour l'opération de stérilisation et le procédé étant réalisé au moins partiellement sous vide.

2. Procédé selon la revendication 1, **caractérisé en ce que** le peroxyde d'hydrogène est vaporisé sous pression ambiante et introduit dans l'article (100) à température avec un milieu porteur, le milieu porteur étant de préférence de l'air, notamment de l'air stérile (L).

3. Procédé selon l'une quelconque des revendications précédentes, **caractérisé en ce que** la température de l'agent de stérilisation (40) est située dans la plage comprise entre 40 °C à 100 °C.

4. Procédé selon l'une quelconque des revendications précédentes, **caractérisé en ce que** la durée du procédé est comprise entre 3 à 20 minutes, notamment est de 5 minutes.

5. Procédé selon la revendication 4, **caractérisé en ce que** le ou les moyens de fermeture sont ou comprennent un ou plusieurs capuchons de fermeture.

6. Système comprenant au moins un dispositif de stérilisation (10) ainsi qu'au moins un article (100) à stériliser par le dispositif de stérilisation (10), le dispositif de stérilisation (10) comprenant au moins une conduite d'alimentation (80, 82, 84) et au moins une conduite d'évacuation (90) pour l'agent de stérilisation (40), l'article (100) présentant au moins deux ouvertures (110, 112, 114, 116) vers un espace intérieur à stériliser de l'article (100), au moins une première ouverture (110, 112, 114) étant raccordée à une conduite d'alimentation (80, 82, 84) pour l'agent de stérilisation (40) et l'au moins une deuxième ouverture (116) étant raccordée à une conduite d'évacuation (90) pour l'agent de stérilisation (40) et le dispositif de stérilisation (10) présentant des moyens au moyen desquels l'agent de stérilisation (40) peut être conduit dans l'espace intérieur et à travers l'espace intérieur par la conduite d'alimentation (80, 82, 84) et au moyen desquels l'agent de stérilisation (40) peut être évacué de l'espace intérieur par la conduite d'évacuation (90), l'agent de stérilisation contenant du peroxyde d'hydrogène ou consiste en peroxyde d'hydrogène, et le peroxyde d'hydrogène pouvant être introduit dans l'article (100) sous forme de vapeur,
**caractérisé en ce que**
des moyens de fermeture sont prévus pour le scellement étanche aux germes de la première ouverture (110, 112, 114) et de la deuxième ouverture (116), le scellement étanche aux germes pouvant être effectué par une fermeture de moyens de fermeture disposés sur l'article dans des conditions stériles, qui forment une partie de la première ouverture (110, 112, 114) et de la deuxième ouverture (116), **en ce que** l'article (100) à stériliser est un ensemble de tubes médicaux, notamment un système de tubes pour le sang, et
**en ce que** le dispositif de stérilisation présente une chambre de réception (200) pour recevoir l'article (100) à stériliser, la chambre de réception (200) étant reliée à une pompe à vide (210) pour faire le vide dans la chambre de réception (200).

7. Système selon la revendication 6, **caractérisé en ce que** le dispositif de stérilisation (10) présente au moins une alimentation en surpression (20), au moins un réservoir (30) pour recevoir l'agent de stérilisation (40), au moins un élément de soupape (50), au moins une alimentation de milieu porteur (60) et au moins un élément de chauffage (70), au moyen desquels l'agent de stérilisation (40), notamment le peroxyde d'hydrogène, peut être vaporisé sous pression ambiante et introduit dans l'article à température avec un milieu porteur, le milieu porteur étant de préférence de l'air, notamment de l'air stérile (L), et l'alimentation de milieu porteur (60) étant de préférence une alimentation en air stérile.

8. Système selon la revendication 6 ou 7, **caractérisé en ce que** le dispositif de stérilisation (10) permet de réaliser un procédé selon l'une quelconque des revendications 1 à 5, le dispositif de stérilisation (10) présentant de préférence un moyen de commande et/ou de régulation au moyen duquel le dispositif de stérilisation (10) peut être commandé et/ou régulé de telle sorte que le procédé selon l'une quelconque des revendications 1 à 5 peut être réalisé et/ou **en ce que**, notamment au moyen du moyen de commande et/ou de régulation, la pression et/ou la température et/ou la composition de l'agent de stérilisation (40) peuvent être commandées et/ou régulées.

9. Utilisation d'un système selon l'une quelconque des revendications 6 à 8 pour la stérilisation d'au moins un article (100), consistent en un ensemble de tubes médicaux, notamment d'un système de tubes pour le sang.
